# EUROPÄISCHE PATENTSCHRIFT

(11) **EP 3 031 981 B1**
(45) Veröffentlichungstag und Bekanntmachung des Hinweises auf die Patenterteilung: **07.02.2018**
(21) Anmeldenummer: 15197917.6
(22) Anmeldetag: 04.12.2015
(51) Int. Cl.: D21F 7/00, D21G 9/00, G01N 21/89

(54) **VERFAHREN ZUR MARKIERUNG EINER FEHLERSTELLE BEI DER HERSTELLUNG EINER PAPIERBAHN**
METHOD FOR MARKING A FAULT LOCATION IN THE PRODUCTION OF A PAPER SHEET
PROCEDE DE MARQUAGE D'UNE ERREUR LORS DE LA FABRICATION D'UNE BANDE DE PAPIER

(30) Priorität: 10.12.2014 DE 102014118346
(43) Veröffentlichungstag der Anmeldung: 15.06.2016
(73) Patentinhaber: Schoeller Technocell GmbH & Co. KG, 49086 Osnabrück (DE)
(72) Erfinder: VON DEN HOFF, Frank, 04249 Leipzig (DE); HINDELANG, Florian, 87437 Kempten (DE); WACHTER,Robert, 87600 Kaufbeuren (DE)
(74) Vertreter: Cohausz & Florack

(56) Entgegenhaltungen:
- WO-A1-2012/012814
- DE-A1-102009 029 083

## Beschreibung

### TECHNISCHES GEBIET DER ERFINDUNG

Die Erfindung betrifft ein Verfahren zur Markierung von Fehlstellen in einer Papierbahn bei der Erzeugung der Bahn in einer Papiermaschine und eine Vorrichtung oder Anordnung zur Durchführung dieses Verfahrens.

### HINTERGRUND DER ERFINDUNG

Bei der Herstellung einer Papierbahn in einer Papiermaschine wird zunächst eine Suspension von Fasserstoff, beispielsweise Zellstoff, und optional Füll- und Hilfsstoffen, auf ein umlaufendes Rund- oder Langsieb aufgebracht. Auf dem Sieb erfolgt eine teilweise Entwässerung der Faserstoffsuspension und damit die Bildung einer Papierbahn.

Die Breite der Papierbahn wird üblicherweise durch Schneiden der nassen Papierbahn auf dem Sieb mittels "Gautschknecht" durch Wasserstrahlschneiden auf beiden Seiten der Bahn eingestellt. Dabei wird die gewünschte Bahnbreite derart eingestellt, dass nur der mittlere Bereich mit der exakten gewünschten Bahnbreite in die anschließende Pressenpartie der Papiermaschine überführt wird. Die Papierbahn wird dort und im weiteren Verlauf der Maschine weiter entwässert und getrocknet, optional durch ein- oder beidseitiges Auftragen einer Leimflotte und/oder durch Kalandrieren oberflächlich nachbehandelt und zuletzt auf einer Stahlwelle, dem Tambour, aufgerollt.

Bei der Blattbildung auf dem Sieb der Papiermaschine oder im weiteren Verlauf der Papierbahnbehandlung können unbeabsichtigt Fehlstellen in der Papierbahn entstehen. Fehlstellen sind beispielweise Einrisse, Löcher oder auch Verschmutzungen.

Solche Fehlstellen sind visuell oder optisch mit einem Kamera-Bildverarbeitungssystem an beliebiger Stelle im Bahnverlauf der Papiermaschine detektierbar. Fehlstellen können während der Blattbildung auf dem Sieb aber auch im weiteren Verlauf nach der Trocknung und/oder Oberflächenbehandlung der Bahn erzeugt werden. Eine Fehlerdetektion im Verlauf der Erzeugung der Papierbahn in der Papiermaschine hat den Vorteil, dass auch Fehlstellen, die während der Trocknung und gegebenenfalls Oberflächenbehandlung der Bahn entstehen, mit erkannt werden. Für die Weiterverarbeitung der an der Papiermaschine erzeugten Papierrolle ist es wünschenswert, mögliche Fehlerbereiche und deren Position in der Rolle einfach und leicht erkennbar anzuzeigen.

In der DE 10 2009 029083 A1 und der DE 10 2009 029084 A1 wird vorgeschlagen, die optisch detektierten Fehlstellen in einer Papierbahn mit Angaben zur Wickellänge der erzeugten Papierrolle bis zur Fehlstelle in einem Datenspeicher, gegebenenfalls auch mit Zusatzinformationen zur Art der Fehlstelle, abzuspeichern und bei der Weiterverarbeitung der Papierrolle zur Verfügung zu stellen. Nachteilig ist an diesem Verfahren aber, dass das Vorhandensein und die Position einer oder mehrerer Fehlstellen in einer Papierrolle vom Bedienungspersonal nicht einfach und ohne weitere Hilfsmittel erkannt werden kann.

Für die Markierung von Fehlstellen in einer Bahnpapierbahn wird in der DE 10 2009 029 081 A1 vorgeschlagen, eine Lasermarkierung auf der Papierbahn anzubringen, die bei der Weiterverarbeitung erkannt und ausgewertet wird. Auch hier ist es aber bei einer aufgerollten Papierbahn nicht erkennbar, ob und gegebenenfalls wo in der Rolle Fehlstellen vorliegen.

Die WO 2012/012814 A1 betrifft eine Anlage zur Herstellung einer Papier- oder Zellstoffbahn mit einem Entwässerungssieb, einer Saugwalze und einem Trockner, wobei zur Bahnbreitenbegrenzung vor der Saugwalze Randspritzdüsen vorgesehen sind. In dieser Anlage sind die Randspritzdüsen derart verstellbar, dass die Bahnbreite in einer laufenden Maschine manuell direkt oder über ein computergesteuertes Programm auf eine vorbestimmte Bahnbreite erfolgen kann.

Aus der DE 10 2014 205 504 A1 ist eine Maschine zur Herstellung oder Veredelung einer Faserstoffbahn bekannt, in der die Faserstoffbahn mit einer Trenneinrichtung in Teilbahnen getrennt wird und die Trenneinrichtung eine Markiervorrichtung zur Markierung einer an die Trenneinrichtung angrenzenden Teilbahn aufweist. Die DE 10 2014 205 504 A1 betrifft ein Verfahren und eine Anordnung zur Reduzierung von Bahnabrissen in der Herstellung von Papier oder Karton.

In der Praxis werden Fehlstellen in aufgerollten Bahnen auch durch aufgeklebte überstehenden Etiketten oder Fähnchen markiert. Nachteilig an solchen Systemen ist aber, dass für das automatische Einbringen und Aufkleben der Etiketten oder Fähnchen eine zusätzliche aufwändige Vorrichtung erforderlich ist und dass Etiketten oder Fähnchen als zusätzliches Material bereitgestellt werden müssen.

### ZUSAMMENFASSUNG DER ERFINDUNG

Der Erfindung liegt folglich Aufgabe zugrunde, ein einfach auszuführendes Verfahren zur Markierung von Fehlstellen in einer auf einer Papiermaschine hergestellten Papierbahn bereit zu stellen. Das Vorhandensein von Fehlstellen und gegebenenfalls deren Position in der Papierrolle sollen dabei ohne weitere Hilfsmittel erkennbar sein.

Gelöst wird diese Aufgabe durch ein Verfahren zur Markierung von Fehlstellen in einer Papierbahn bei der Erzeugung der Bahn in einer Papiermaschine, in dem unmittelbar nach Ermittlung einer Fehlstelle in der Papierbahn durch eine Fehlerdetektionseinrichtung über eine Steuereinheit die Position von mindestens einer Wasserstrahldüse zur Begrenzung der Bahnbreite der Papierbahn in der Siebpartie der Papiermaschine temporär derart geändert wird, dass kurzzeitig eine mindestens einseitig breitere Papierbahn erzeugt wird.

Beschrieben wird ferner eine Einrichtung oder Anordnung zur Durchführung des erfindungsgemäßen Verfahrens. Diese Anordnung umfasst, ein Fehlerdetektionssystem, eine Auswertungseinheit und eine Steuerungseinheit zur Positionsänderung von einer oder mehrerer Wasserstrahldüsen zur Begrenzung der Bahnbreite der Papierbahn in Abhängigkeit vom gelieferten Signal.

Die Verfahrensweise hat den Vorteil, dass selbst an der Papierrolle durch das Überstehen von Bahnanteilen leicht festgestellt werden kann, wo sich eine Fehlstelle in der Papierbahn befindet.

### BESCHREIBUNG DER BEVORZUGTEN AUSFÜHRUNGFORMEN

Das erfindungsgemäße Verfahren kann bei der Herstellung von bahnförmigen Materialien für alle Papierspezialitäten, Kartons oder Pappen angewendet werden. Fehlstellen nach der Blattbildung in der Papierbahn werden automatisiert festgestellt. Die Information über das Vorhandensein einer Fehlstelle an der Bahn wird an eine Steuerungseinheit weitgeleitetet. Die Steuerungseinheit veranlasst Wasserstrahldüsen zur seitlichen Begrenzung oder Breiteneinstellung der Papierbahn zur Positionsänderung. Die Positionsänderung der Wasserstrahldüsen führt dazu, dass die Papierbahn kurzfristig an Breite zunimmt. Die Zunahme an Breite kann einseitig oder beidseitig erfolgen. Die Papierbahn erstreckt sich nach der Positionsänderung zumindest einseitig über die ansonsten eingestellte Bahnbreite hinaus. Die Zunahme der Breite der Papierbahn ist temporär. Diese Markierungen der Papierbahn durch eine vergrößerte Bahnbreite sind auch nach dem Aufwickeln oder Aufrollen der Bahn visuell noch erkennbar. Sie erlauben ein leichtes Auffinden der Bahnposition, wo eine Fehlstelle vorhanden und zuvor detektiert worden ist.

Durch die Wasserdüsen wird ein zum Schneiden oder Begrenzen der Papierbahn geeigneter Wasserstrahl erzeugt. Diese werden auch als "Gautschknechte" bezeichnet. Das Schneiden mit Wasserstrahl erfolgt in der Siebpartie der Papiermaschine. Nach Fehlerdetektion kann erfindungsgemäß die Position der Düse und damit die Schneidposition in der Papierbahn vorübergehend so verstellt werden, dass kurzzeitig eine geringfügig breitere Papierbahn erzeugt wird. Nach dem Entwässern, Trocknen, der optionalen üblichen Nachbehandlung der Papierbahn und deren abschließendem Aufrollen ist somit das Vorhandensein einer Fehlstelle und deren Position in der Papierrolle durch ein seitlich überstehendes Blattsegment in Form eines Fähnchens einfach zu erkennen oder zu ermitteln.

In einer bevorzugten Ausführungsform der Erfindung erfolgt die Positionsänderung der Wasserstrahldüse nur auf einer Seite der Papierbahn bei Detektion einer Fehlstelle in der Papierbahn derart, dass die Düse für einen Zeitdauer von 0,01 bis 10 Sekunden um eine Strecke von 2 mm bis 100 mm, besonders bevorzugt 5 mm bis 20 mm quer zur Laufrichtung der Papierbahn nach außen verstellt wird. So wird temporär, also für einen begrenzten Zeitraum, eine größere Bahnbreite erzeugt. Die Länge der erzeugten Markierungsfahne in Maschinenrichtung ergibt sich aus dem Produkt der Maschinengeschwindigkeit (Bahngeschwindigkeit) und der Zeit, für die die Position der Wasserstrahldüse geändert ist. Die Länge der Markierungsfahne beträgt vorzugsweise 0,01 m bis 10 m.

Die Positionsänderung von mindestens einer Wasserstrahldüse oder zwei Wasserstrahldüsen kann in einer bevorzugten Ausführungsform dadurch erfolgen, dass die Halterung der Düse auf einer schlittenförmigen Führungseinheit angeordnet ist und diese zum Beispiel mit einem Pneumatik-Zylinder kurzeitig in die Position der größeren Bahnbreite der Papierbahn verfahren wird. Alternativ kann die Positionsänderung auch durch einen Hubmagneten oder einen anderen Antrieb erfolgen.

Das Steuersignal zur Positionsänderung der Wasserstrahldüse (n) kann direkt von der Fehlerdetektionseinheit weitergegeben werden. Vorzugsweise ist die Fehlerdetektionseinheit oder das Inspektionssystem ein Kamera-Inspektionssystem. Dieses kann in der Papiermaschine angeordnet sein. Es kann unter oder oberhalb der Papierbahn positioniert sein, vorzugsweise oberhalb der Papierbahn. Dieses wertet durchgehend die Oberfläche der Papierbahn aus. Beim Auftreten einer Unregelmäßigkeit in der Papierbahn wird ein Fehlersignal erzeugt. In Abhängigkeit von der Erzeugung eines Fehlersignals wird die Position von einer oder zwei Wasserstrahldüsen verändert mit der Folge, dass die Papierbahn temporär bereiter wird. Fehlstellen in der Papierbahn können auch über andere geeignete physikalische Messgrößen ermittelt, für die Erkennung einer Fehlstelle in der Bahn und Auslösung der Steuersignalsignals zur Positionsänderung der Wasserstrahldüse herangezogen werden, wie Druck- oder Durchflussänderungen des vom Sieb anlaufenden Siebwassers, Änderungen des elektrischen Wiederstands der Papierbahn oder der Durchlässigkeit der Papierbahn für elektromagnetische oder radioaktive Strahlung. Diese Änderungen können an jeder Stelle des Stoff- oder Bahnlaufs in der Papiermaschine detektiert werden. Vorzugsweise kann die Detektion einer Fehlstelle der Papierbahn kurz vor dem Aufrollen der Papierbahn erfolgen.

Abhängig vom Ort der Fehlerdetektion in der Papiermaschine und der Verarbeitungszeit des Fehlersignals kann die erfindungsgemäße Fehlstellenmarkierung durch die erhöhte Bahnbreite im Verlauf der Papierbahn weniger als 1 m bis mehr als 1000 m hinter der Fehlstelle liegen. Diese Entfernung ist eine von der Bahngeschwindigkeit, der Bahnlänge, gemessen von der Wasserstrahldüse bis zu der Detektionsstelle, und der Signalverarbeitungsdauer abhängige und somit festliegende Größe, so dass von der Markierung der Papierbahn durch die erhöhte Bahnbreite beginnend die Lage der Fehlstelle eindeutig ermittelt werden kann. Da die Markierung ohne weitere Hilfsmittel an der Stirnseite oder den Stirnseiten der Rolle erkannt werden kann, kann bei dem Abrollen der Papierrolle die Fehlstelle leicht aufgefunden und gegebenenfalls entfernt und verworfen werden.

Dass sich durch die Auswertung der Daten zur Papierbahn ergebende Fehlersignal wird herangezogen zur Steuerung oder zur Erzeugung eines Steuersignals für die seitliche Begrenzungseinrichtung, insbesondere die Wasserstrahldüsen. Die Erzeugung des Steuersignals für die Wasserstrahldüsen kann in der Fehlerdetektionseinheit oder in der Steuerungseinrichtung selbst vorgenommen werden.

Fig. 1 zeigt eine schematische Darstellung des erfindungsgemäßen Verfahrens.

Eine Ausführungsform der Erfindung wird nachfolgend unter Berücksichtigung der Fig. 1 weiter erläutert. Der Faserbrei wird zur Blattbildung auf das Sieb 1 der Papiermaschine aufgetragen, wo die Bildung der Papierbahn 2 durch das Entfernen von Wasser erfolgt. Die durch Wasserstrahldüsen 4, 4' erzeugten Wasserstrahlen begrenzen die Papierbahn auf die durch 8 und 8' gekennzeichneten Bahnränder. Nach Detektion der Fehlstelle 3 auf der Papierbahn 2 durch ein Inspektionssystem 6 wird ein Fehlersignal über die Datenleitung 7 an die Steuereinheit 5 geleitet. Die Steuereinheit 5 führt eine kurzfristige Positionsänderung der Wasserstrahldüse 4' durch. Die Wasserstrahldüse 4', die zuvor die Papierbahn 2 auf den seitlichen Rand 8 begrenzt hatte, schneidet die Papierbahn dann durch die Positionsänderung in Position 9, wodurch die Papierbahn an dieser Stelle verbreitert wird. Anschließend wir die Wasserstrahldüse 5 wieder in die zuvor eingestellte Position 8' zurückgefahren, wodurch sich bei Position 9 eine Fahne ausbildet.

## Patentansprüche

1. Verfahren zur Markierung von Fehlstellen in einer Papierbahn bei der Erzeugung der Bahn an einer Papiermaschine, **dadurch gekennzeichnet, dass** unmittelbar nach Ermittlung einer Fehlstelle in der Papierbahn durch eine Fehlerdetektionseinheit die Position von mindestens einer Wasserstrahldüse zur seitlichen Begrenzung der Papierbahn in der Siebpartie der Papiermaschine temporär derart geändert wird, dass kurzzeitig eine mindestens einseitig breitere Papierbahn erzeugt wird.

2. Verfahren nach Anspruch 1, **dadurch gekennzeichnet, dass** die breitere Papierbahn durch Änderung der Position der Wasserstrahldüsen zur seitlichen Begrenzung der Papierbahn über eine Zeitspanne von 0,01 Sekunden bis 10 Sekunden erfolgt.

3. Verfahren nach einem der Ansprüche 1 bis 2, **dadurch gekennzeichnet, dass** die kurzzeitige Verbreiterung der Papierbahnbreite 2 bis 100 mm in Maschinenrichtung der Bahn beträgt.

4. Verfahren nach einem der Ansprüche 1 bis 3, **dadurch gekennzeichnet, dass** die Wasserstrahldüsen zur Begrenzung der Papierbahnbreite elektromechanisch oder pneumatisch verstellt werden.

5. Verfahren nach Anspruch 4, **dadurch gekennzeichnet, dass** das Signal zur Änderung der Position von mindestens einer Wasserstrahldüse durch ein Kamera-Bahninspektionssystem veranlasst oder erzeugt wird.

## Claims

1. Method for marking defects in a paper web during the production of the web on a paper machine, **characterised in that,** immediately after a defect in the paper web is detected by a defect detection unit, the position of at least one water jet nozzle defining the lateral boundaries of the paper web in the wire section of the paper machine is temporarily changed such that for a brief time a paper web which is wider on at least one side is produced.

2. Method according to Claim 1, **characterised in that** the widening of the paper web by changing the position of the water jet nozzles defining the lateral boundaries of the paper web takes place over a period of time of 0.01 seconds to 10 seconds.

3. Method according to any one of Claims 1 to 2, **characterised in that** the brief widening of the width of the paper web amounts to 2 to 100 mm in the machine direction of the web.

4. Method according to any one of Claims 1 to 3, **characterised in that** the water jet nozzles limiting the width of the paper web are adjusted electromechanically or pneumatically.

5. Method according to Claim 4, **characterised in that** the signal to change the position of at least one water jet nozzle is initiated or generated by a camera web inspection system.

## Revendications

1. Procédé de marquage d'un défaut dans une bande de papier lors de la fabrication de la bande sur une machine à papier,
**caractérisé en ce que,** immédiatement après qu'un défaut ait été détecté dans la bande de papier par une unité de détection de défauts, la position d'au moins une buse à jet d'eau, limitant latéralement la bande de papier, est modifiée temporairement, dans la partie de tamisage de la machine à papier, de manière à ce qu'une bande de papier plus large, au moins sur un côté, soit produite pendant une courte durée.

2. Procédé selon la revendication 1,
**caractérisé en ce que** la bande de papier plus large est obtenue par modification de la position des buses à jet d'eau, qui limitent la largeur de la bande de papier, pendant un laps de temps de 0,01 seconde à 10 secondes.

3. Procédé selon l'une des revendications 1 à 2,
**caractérisé en ce que** l'élargissement de courte durée de la largeur de la bande de papier est de 2 à 100 mm dans le sens de déplacement de la bande dans la machine.

4. Procédé selon revendication 1 à 3,
**caractérisé en ce que** les buses à jet d'eau sont réglées par commande électromécanique ou pneumatique.

5. Procédé selon la revendication 4,
**caractérisé en ce que** le signal de modification de la position d'au moins une buse à jet d'eau est incité ou généré par un système d'inspection de bande à caméra.
